# EUROPEAN PATENT SPECIFICATION

(11) **EP 3 755 813 B1**
(45) Date of publication and mention of the grant of the patent: **09.08.2023**
(21) Application number: 19708786.9
(22) Date of filing: 19.02.2019
(51) Int. Cl.: C12Q 1/686, C12Q 1/6886

(54) **IMPROVED DETECTION OF MICROSATELLITE INSTABILITY**
VERBESSERTE DETEKTION VON MIKROSATELLITENINSTABILITÄT
DÉTECTION AMÉLIORÉE DE L'INSTABILITÉ MICROSATELLITAIRE

(30) Priority: 20.02.2018 US 201862632910 P
(43) Date of publication of application: 30.12.2020
(73) Proprietor: F. Hoffmann-La Roche AG, 4070 Basel (CH); Roche Diagnostics GmbH, 68305 Mannheim (DE)
(72) Inventor: LITTERST, Claudia, Pleasanton, California 94588 (US); TRAN, Ha, Bich, Pleasanton, California 94588 (US); YANG, Wei, Pleasanton, California 94588 (US)
(74) Representative: Hövelmann, Sascha Alexander
(86) International application number: PCT/EP2019/054013
(87) International publication number: WO 2019/162240

(56) References cited:
- WO-A1-2019/011971
- KR-A- 20170 037 095
- CURTIS B. HUGHESMAN ET AL: "A Robust Protocol for Using Multiplexed Droplet Digital PCR to Quantify Somatic Copy Number Alterations in Clinical Tissue Specimens", PLOS ONE, vol. 11, no. 8, 18 August 2016 (2016-08-18), page e0161274, XP055590753, DOI: 10.1371/journal.pone.0161274 -& Curtis B Hughesman: "A Robust Protocol for Using Multiplexed Droplet Digital PCR to Quantify Somatic Copy Number Alterations in Clinical Tissue Specimens - S1 Table", , 18 August 2016 (2016-08-18), XP055590761, Retrieved from the Internet: URL:https://journals.plos.org/plosone/arti cle/file?type=supplementary&id=info:doi/10 .1371/journal.pone.0161274.s008 [retrieved on 2019-05-21]
- LIU TIANFU ET AL: "A novel real-time reverse transcription-polymerase chain reaction assay with partially double-stranded linear DNA probe for sensitive detection of hepatitis C viral RNA", JOURNAL OF VIROLOGICAL METHODS, ELSEVIER BV, NL, vol. 236, 20 July 2016 (2016-07-20), pages 132-138, XP029710180, ISSN: 0166-0934, DOI: 10.1016/J.JVIROMET.2016.07.015
- DIETMAIER W ET AL: "DETECTION OF MICROSATELLITE INSTABILITY BY REAL TIME PCR AND HYBRIDIZATION PROBE MELTING POINT ANALYSIS", LABORATORY INVESTIGA, NATURE PUBLISHING GROUP, THE UNITED STATES AND CANADIAN ACADEMY OF PATHOLOGY, INC, vol. 81, no. 10, 1 October 2001 (2001-10-01), pages 1453-1456, XP009050845, ISSN: 0023-6837

## Description

### BACKGROUND OF THE INVENTION

Microsatellites are repetitive regions with a length of 1-6 nucleotides dispersed throughout the genome. Microsatellite instability (MSI) manifests as alterations in the repeat length, namely expansion or contraction, in one or both alleles. MSI is caused by defects in the mismatch repair system, either due to germline mutations (Lynch Syndrome) or due to sporadic somatic genetic or epigenetic changes in the mismatch repair genes. MSI is a valuable diagnostic tool for Lynch Syndrome screening because tumors of almost all patients with mutations in a mismatch repair gene have an MSI phenotype.

MMR-deficiency is associated with a large proportion of mutant neoantigens in cancers which make them sensitive to immune checkpoint blockade, regardless of the tissue of origin (Le et al. (2017) Science 357:409). Therefore, MSI is a predictive biomarker for immune checkpoint therapy response in multiple cancers, e.g., glioblastoma, colorectal, endometrial, ovarian, gastric, prostate, and breast cancer. The FDA has granted accelerated approval to Pembrolizumab (Keytruda) for pediatric and adult patients with MMR deficient solid tumors. In early stage CRC, patients with dMMR have better survival rates, indicating that MSI also has prognostic value (Merok et al. (2013) Annals Oncology 24: 1274; Roth et al. (2012) J. Natl. Cancer Inst. 104:1635). KR20170037095A (SEASUNBIO MAT [KR]) describes a method for diagnosing microsatellite instability using molecular beacon drop-off probes. Hughesman ET AL (PLOS ONE, vol. 11, no. 8 (2016-08-18), e0161274) includes the use of a reference probe as an internal control, albeit not to a region near the microsatellite repeat.

### SUMMARY OF THE INVENTION

Provided herein are oligonucleotides, kits, assays, and methods for detecting microsatellite instability (MSI). The invention is set out in the appended set of claims.

In one aspect kits for detecting microsatellite instability, if present, in a sample are provided comprising genomic DNA from an individual, *e.g.*, a tumor sample from a human. Herein, the kit comprises: a) a drop-off probe having a sequence complementary to a wild type microsatellite repeat sequence in genomic DNA with at least one additional complementary nucleotide (*e.g.*, 1, 2, 3, 4, 5, 6, 3-7, 7, 8, 9, 10, or more) on the 5' end and at least one additional complementary nucleotide (*e.g.*, 1, 2, 3, 4, 5, 6, 3-7, 7, 8, 9, 10, or more) on the 3' end of the wild type microsatellite repeat sequence, wherein the drop-off probe is attached to a first label (*e.g.*, fluorophore) and a quencher; b) a reference probe having a sequence that is complementary to a reference sequence in genomic DNA that is not microsatellite repeat sequence, wherein the reference probe is attached to a second label (*e.g.*, fluorophore) and a quencher; and a set of amplification primers positioned 5' and 3' of the binding sites of the drop-off probe and reference probe, said set of amplification primers being designed to amplify both binding sites of the drop-off probe and the reference probe.

In some embodiments, and depending on the microsatellite to be detected, the drop-off probe is at least 10-80 or 25-50 nucleotides in length. For example, the drop-off probe is the length of the normal, most common microsatellite repeat sequence, plus the additional nucleotides on the 5' and 3' ends of the drop-off probe that are complementary to the genomic sequence adjacent to the microsatellite repeat sequence. In some embodiments, the reference probe is similar in size, or has a similar melting temperature to the drop-off probe.

In some embodiments, the reference probe binds to a conserved / invariant genomic sequence near the drop-off probe, *e.g.*, within 1-5, 5-10, 10-50, 50-100, 100-500 nucleotides from the binding site of the drop-off probe.

In some embodiments, the kit further comprises a thermostable DNA polymerase. In some embodiments, the kit further includes controls, *e.g.*, DNA with a known microsatellite instability (positive control), or with a normal (wild type) microsatellite sequence (negative control).

The quencher on the drop-off probe is attached internally on the drop-off probe, *e.g.*, internal to the microsatellite repeat sequence. In some embodiments, the quencher is attached within 4, 5, 6, 7, 8, 9, or 10nucleotides of the first label. In some embodiments, the drop-off probe has an internal quencher, and at least 4, 5, 6, 7, 8, 9, or 10 additional complementary nucleotides on the 5' end of the microsatellite repeat sequence.

In some embodiments, the kit further includes a quencher oligonucleotide complementary to at least a portion of the drop-off probe, wherein the quencher oligonucleotide comprises a quencher. In some embodiments, the quencher is positioned at the 3' end or within five nucleotides of the 3' end of the quencher probe. In some embodiments, the melting temperature of the quencher probe and drop-off probe (temperature at which 50% of the double stranded complex melts into single stranded DNA) is 20-60°C, in particular 42-55°C, 46-52°C or about 50°C. In some embodiments, the melting temperature is less than the temperature selected for annealing in the PCR reaction. In some embodiments, the quencher oligonucleotide is contiguous with the reference probe, so that the reference probe includes some sequence that is not complementary to the microsatellite repeat sequence (*e.g.*, adjacent to the microsatellite repeat sequence), and some sequence that some that is complementary. In some embodiments, the reference probe includes at least 6-20, 8-15, 5, 6, 7, 8, 9, 10, or more nucleotides complementary to the drop-off probe (quencher oligonucleotide portion), with a quencher near the 3' end.

In some embodiments, the drop-off probe forms a hairpin structure at 20-60°C, below 60°C, below 55° or below 50°C, or below the temperature selected for annealing in the PCR reaction. In some embodiments, the drop-off probe includes at least one mismatch with the genomic sequence adjacent to the microsatellite repeat sequence to improve hairpin formation (*i.e*., increase complementarity of nucleotides 5' and 3' of the sequence complementary to the microsatellite repeat sequence).

In some embodiments, the kit includes probes to detect multiple microsatellite repeat sequences. In some embodiments, the kit includes at least 2, 3, 4, 5, 6, 7, 8, 9 or 10 drop-off probes, as described above and herein, having sequences complementary to different wild type microsatellite repeat sequences in genomic DNA (multiple drop-off probes). In some embodiments, the multiple drop-off probes are included in separate vessels, or combined, *e.g.*, with 2, 3, 4, or more drop-off probes in the same vessel. In some embodiments, the multiple drop-off probes are each attached to the same label (*e.g.*, labels with the same fluorescence) and the corresponding multiple reference probes are attached to different labels. In such cases, the signal from the reference probes distinguishes the signal for each microsatellite repeat sequence being detected in the multiplex reaction. In some embodiments, the multiple drop-off probes are attached to labels that are different from the other drop-off probes in the same vessel, or different from the other drop-off probes in the same kit. In some embodiments, the kit further includes multiple reference probes, and/ or multiple primer sets, as described above and herein, paired with each of the multiple drop-off probes. In some embodiments, the multiple reference probes are attached to labels that are different from the other reference and drop-off probes in the same vessel, or different from the other reference and drop-off probes in the same kit.

In a further aspect, reaction mixtures for detecting microsatellite instability, if present in the genomic DNA from an individual, *e.g.*, in a tumor sample are provided. Herein, the reaction mixture comprises a drop-off probe having a sequence complementary to a wild type microsatellite repeat sequence in genomic DNA with at least one nucleotide on the 5' end and at least one nucleotide on the 3' end of the wild type microsatellite repeat sequence, wherein the drop-off probe is attached to a first label and a quencher and wherein the quencher of the drop-off probe is attached at a site within ten nucleotides of the first label; b) a reference probe having a sequence that is complementary to a reference sequence in genomic DNA that is not microsatellite repeat sequence, wherein the reference probe is attached to a second label and a quencher; c) a set of amplification primers positioned 5' and 3' of the binding sites of the drop-off probe and reference probe, said set of amplification primers being designed to amplify both binding sites of the drop-off probe and the reference probe; d) thermostable DNA polymerase; and e) genomic DNA. The drop-off probe and reference probe can include any of the features described above and herein, e.g., an internal quencher, overlapping sequence, etc. In addition, the reaction mixture can be multiplex, with more than one drop-off probe, reference probe, and primer set.

In a further aspect, methods for detecting microsatellite instability if present in a sample of genomic DNA from an individual or group of individuals, *e.g.*, from a tumor sample are provided. Herein, the method comprises a) contacting genomic DNA with the components of the kit as described above and herein; b) carrying out dPCR to amplify and detect binding of the drop-off probe and reference probe to the genomic DNA (*e.g.*, if a positive signal is detected from the first and second label); and c) detecting microsatellite instability when binding of the drop-off probe to genomic DNA is reduced compared to a control or threshold value. In some embodiments, the control value is obtained from normal, non-diseased tissue from the same individual or from a group of individuals (to obtain a broader average value for a population). In some embodiments, the microsatellite instability is detected when binding of the drop-off probe to genomic DNA is reduced and binding of the reference probe remains unchanged, or not reduced as much as binding of the drop-off probe. Herein, the drop-off probe and reference probe can include any of the features described above and herein, *e.g.*, an internal quencher, overlapping sequence, etc. In addition, the methods can be multiplex, with more than one drop-off probe, reference probe, and primer set.

Thus, the method comprises a) contacting genomic DNA with: i) a drop-off probe having a sequence complementary to a wild type microsatellite repeat sequence in genomic DNA with at least one nucleotide on the 5' end and at least one nucleotide on the 3' end of the wild type microsatellite repeat sequence, wherein the drop-off probe is attached to a first label and a quencher and wherein the quencher of the drop-off probe is attached at a site within ten nucleotides of the first label; ii) a reference probe having a sequence that is complementary to a reference sequence in genomic DNA that is not microsatellite repeat sequence, wherein the reference probe is attached to a second label and a quencher; and iii) a set of amplification primers positioned 5' and 3' of the binding sites of the drop-off probe and reference probe, said set of amplification primers being designed to amplify both binding sites of the drop-off probe and the reference probe; b) carrying out dPCR to amplify and detect the binding of drop-off probe and reference probe to genomic DNA, and c) detecting microsatellite instability when binding of the drop-off probe to genomic DNA is reduced compared to a control or threshold value. In some embodiments, the method is carried out in multiplex with 2, 3, 4, 5, or more drop-off probes to detect microsatellite instability at multiple microsatellite repeat sequences.

### BRIEF DESCRIPTION OF THE DRAWINGS

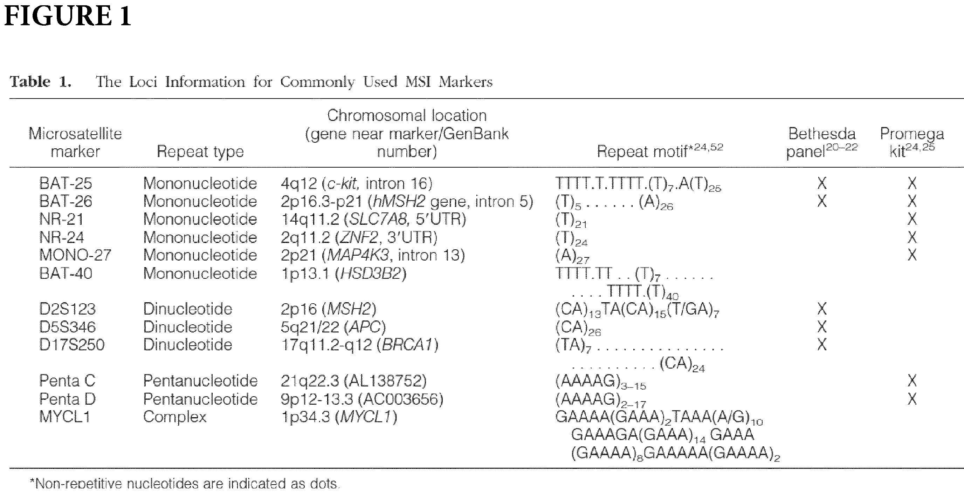

Figure 1 shows a list of commonly detected microsatellites and genomic location. The repeat motif sequences are SEQ ID NO:10 for BAT-25, SEQ ID NO: 11 for BAT-26, SEQ ID NO:12 for NR-21, SEQ ID NO: 13 for NR-24, SEQ ID NO:14 for MONO-27, SEQ ID NO:15 for BAT-40, SEQ ID NO:16 for D2S123, SEQ ID NO:17 for S5S346, SEQ ID NO:18 for D17S250, SEQ ID NO:19 for PentaC, SEQ ID NO:20 for PentaD, and SEQ ID NO:21 for MYCL1.

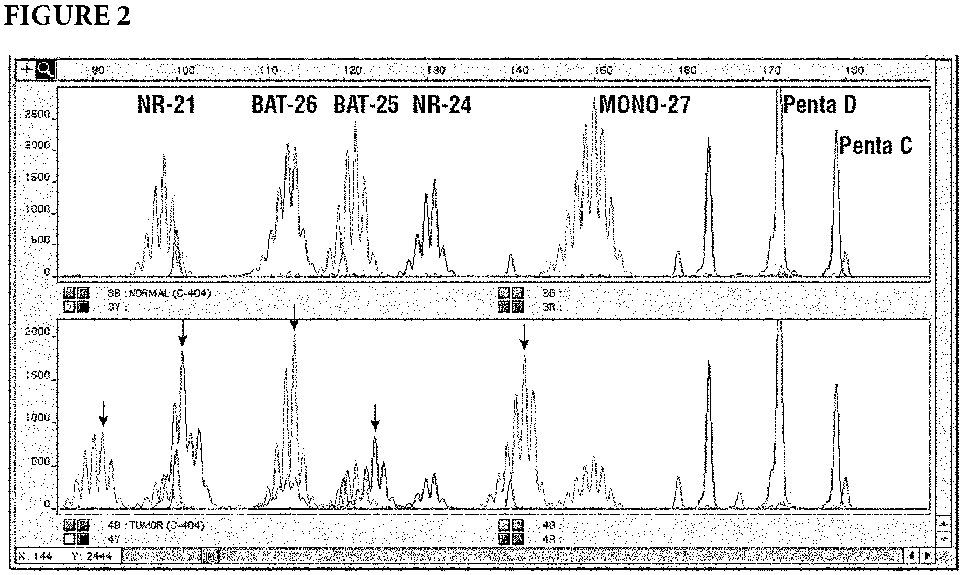

Figure 2 shows an example of results using current methods. Matching normal sample (top panel) and MSI-positive tumor sample (bottom panel) were analyzed using the MSI Analysis System. Two nanograms of genomic DNA was amplified and analyzed using an ABI PRISM 3100 Genetic Analyzer with POP-4 polymer and a 36 cm capillary, and the allelic patterns of the normal and MSI-positive samples are shown. The presence of new alleles in the MSI-positive sample (indicated by arrows) that were not present in the normal sample indicate MSI.

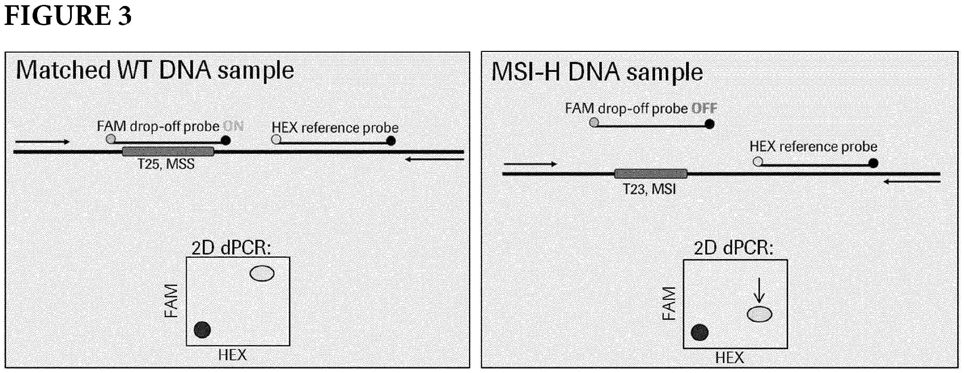

Figure 3 shows an example of a drop-off assay to detect a deletion MSI in marker BAT25. In microsatellite stable sample (MSS), the drop-off probe binds the 25 T/A mononucleotide repeat sequence and some adjacent, "anchor" nucleotides. In the MSI deletion sample (*e.g.*, 23 T/A mononucleotide repeat, the drop-off probe is no longer fully complementary to the genomic sequence, so there is reduced affinity and less fluorescence from hydrolysis of the probe (*i.e.*, hydrolysis resulting in separation of the fluorophore and quencher). In this example, the fluorophore (FAM or HEX) is at the 5' end of the probe, and the quencher is at the 3' end.

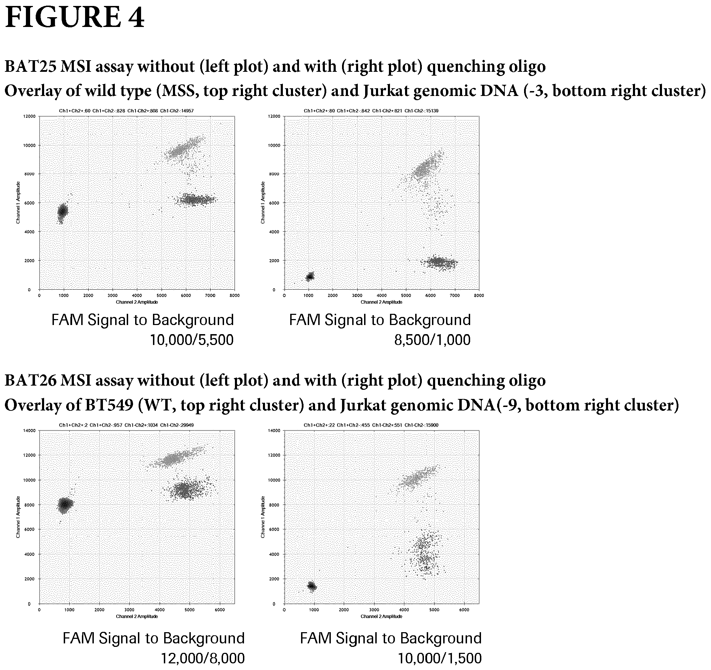

Figure 4 shows dPCR results from a probe design depicted in Figure 3. The left plots show results without a separate quencher oligonucleotide for the BAT25 (top) and BAT26 (bottom) markers. These results show relatively high signal from the drop-off probe fluorophore in the presence of MSI. The right plots show results with a separate quencher oligonucleotide for the BAT25 (top) and BAT26 (bottom) markers. These results show greater separation of MSS and MSI signals due to more effective quenching of the drop-off probe fluorophore.

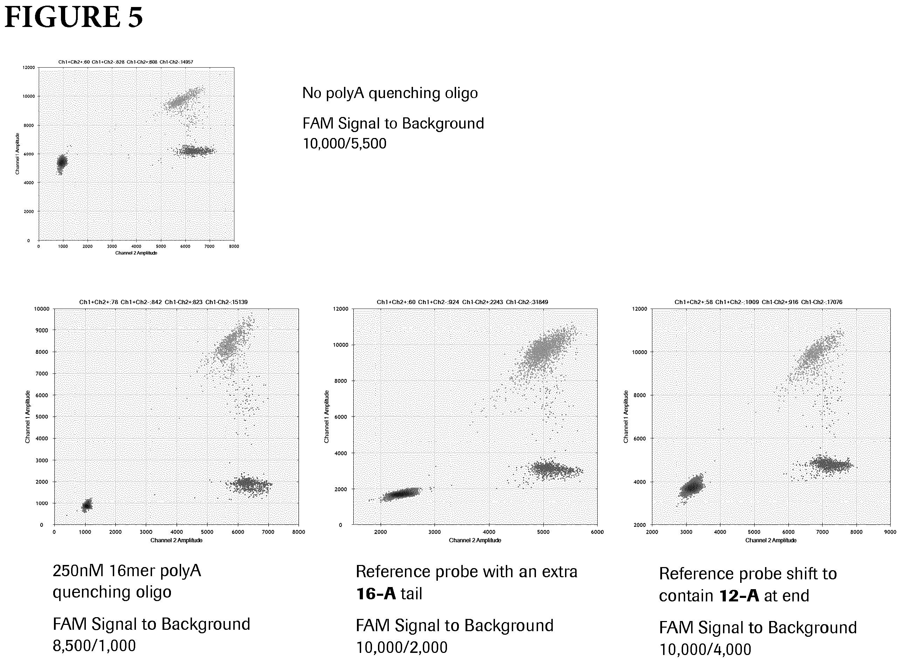

Figure 5 shows comparison of results from a separate 16-mer quencher oligonucleotide to those from quencher oligonucleotide attached to the reference probe. The top plot shows results from an MSI assay lacking quencher oligonucleotide. The bottom left plot shows results from an MSI assay with a separate 16mer quencher oligonucleotide, which as demonstrated in Figure 4, reduces background fluorescence from the non-hydrolyzed drop-off probe. The middle and right bottom plots show that a quencher oligonucleotide attached to the reference probe (16mer and 12mer respectively (SEQ ID NOS 27 and 28, respectively)) are as effective as the separate quencher oligonucleotide.

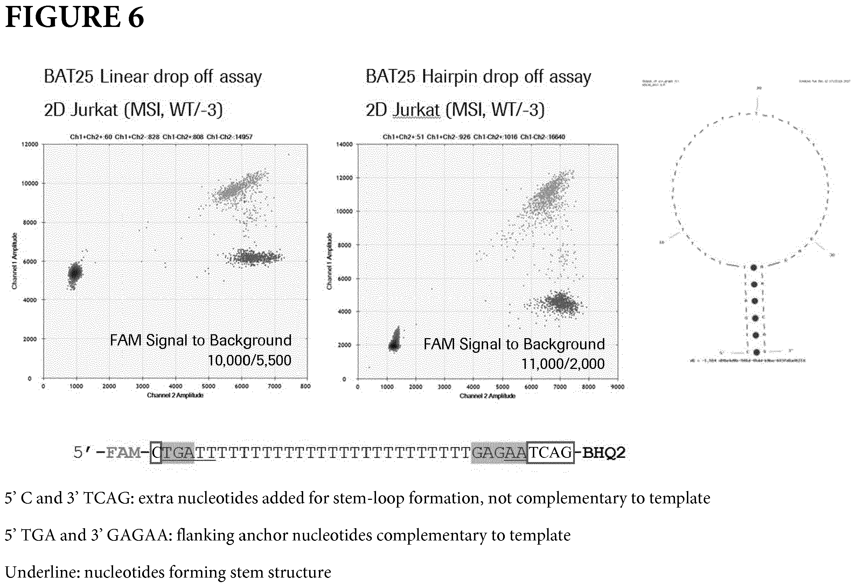

Figure 6 shows design of a drop-off probe that forms a hairpin at 50°C or lower (SEQ ID NO:3). The left plot shows results from a BAT25 MSI assay with a linear drop-off probe (SEQ ID NO: 3) and no quencher oligonucleotide. The right plot shows results from a BAT25 MSI assay using the hairpin drop-off probe. In the hairpin configuration, the quencher and fluorophore are in closer proximity for more effective quenching when the probe is not hydrolyzed (*i.e.*, not bound to genomic microsatellite repeat sequence).

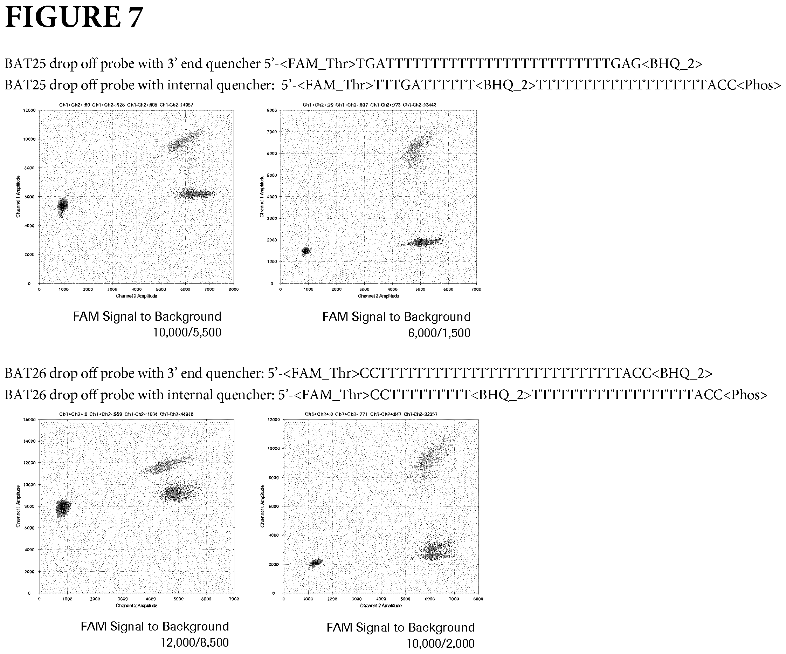

Figure 7 shows drop-off probes with the quencher at different positions relative to the 5' label, *i.e.*, at the 3' end or internal. The drop-off probe for the BAT25 MSI assay is SEQ ID NO:4 (top) and the drop-off probe for the BAT26 MSI assay is SEQ ID NO:5 (bottom). The results show that in both assays, internal placement of the quencher in the drop-off probe is more effective at reducing background fluorescence from unhydrolyzed drop-off probe. Figure 7 also discloses the drop off probe with internal quencher for BAT25 and BAT26 as SEQ ID NOS 22 and 5, respectively.

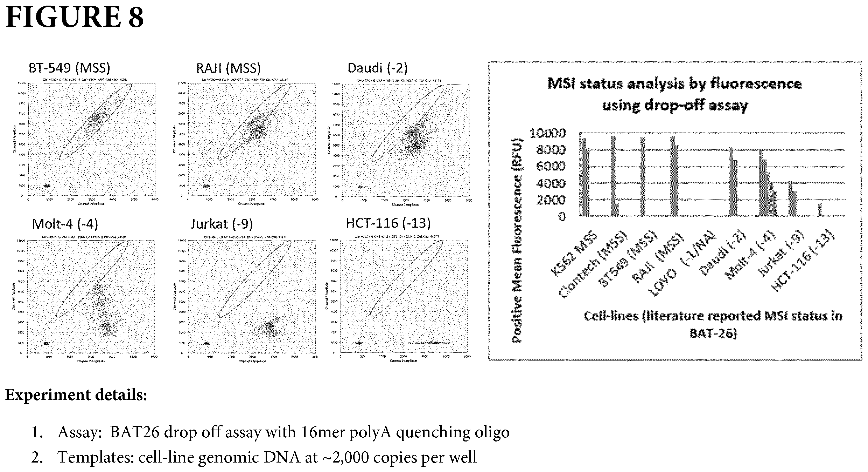

Figure 8 shows that small variations in microsatellite size can be detected using the methods described herein. The plots on the left show results from a BAT26 MSI assay with a quenching oligonucleotide. The MSS status or reported number of nucleotides deleted in MSI cell lines is indicated for the BT-549, Raji, Daudi, Molt-4, Jurkat, and HCT-116 cell lines. The graph on the right shows positive mean fluorescence amplitude for the drop-off probe label (FAM). Some cell lines show multiple results, indicating alleles with different repeat length. Figure 8 discloses "16-mer polyA" as SEQ ID NO: 27.

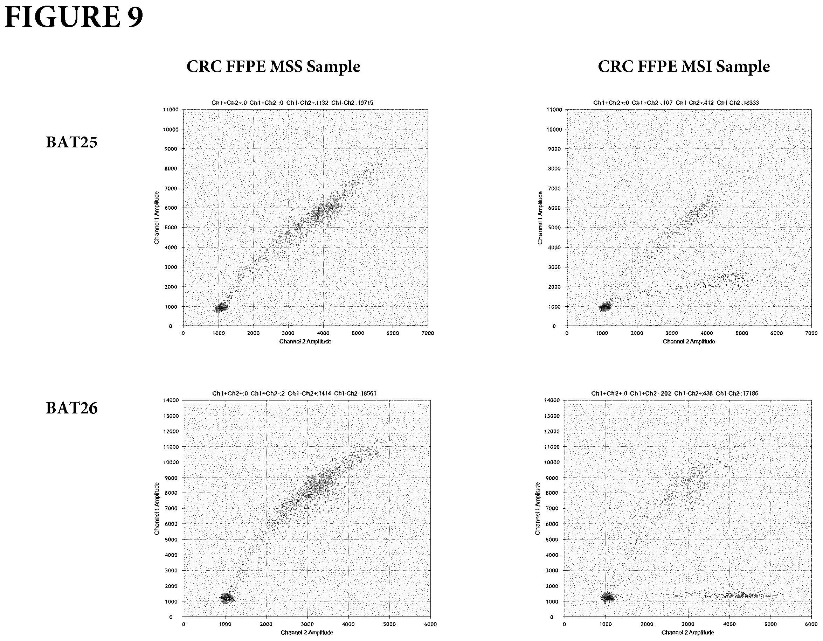

Figure 9 shows that the methods described herein are effective for detecting MSI in DNA from FFPET samples, which is typically damaged and fragmented. The left plots show results from a non-tumor MSS FFPET sample for both BAT25 (top) and BAT26 (bottom) markers, and the right plots show results from tumor MSI FFPET samples. The MSI assays were performed using a quencher oligonucleotide to reduce background fluorescence.

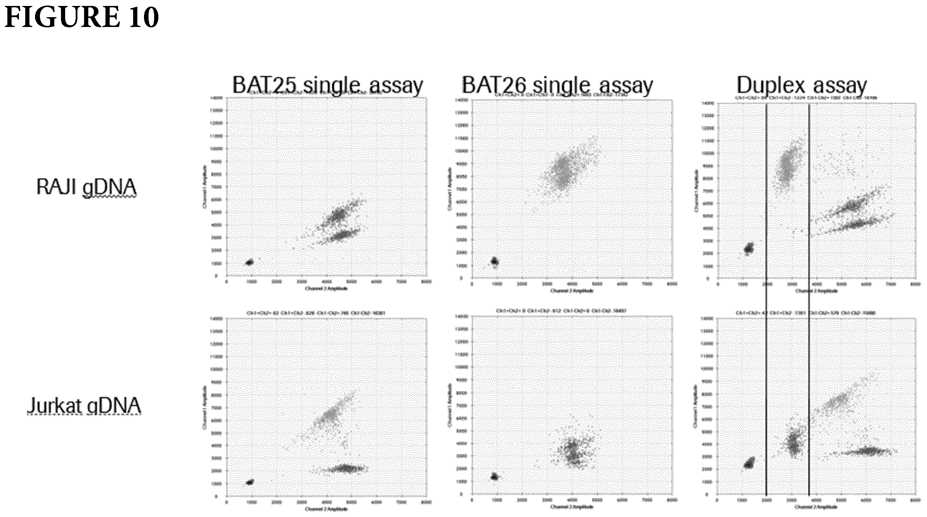

Figure 10 shows that the presently described methods can be multiplexed. The left-most panels show results of the BAT25 MSI assay from two MSI positive cell lines, the middle panels show results of the BAT26 MSI assay, and the right-most panels show results from the multiplexed assays.

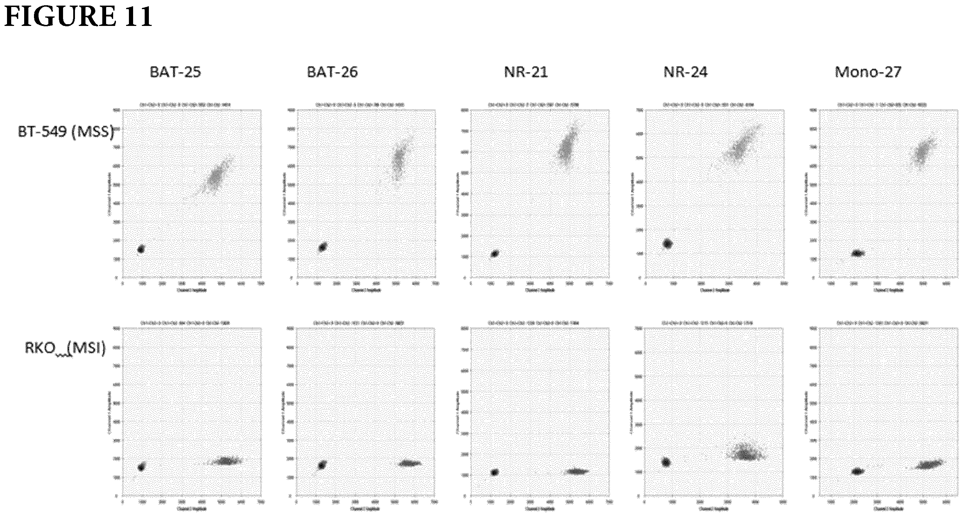

Figure 11 shows results from 2D plots of 5 commonly detected MSI markers in BT-549 (MSS) and RKO (MSI-H) cell lines. The markers are BAT-25, BAT-26, NR-21, NR-24, and Mono-27.

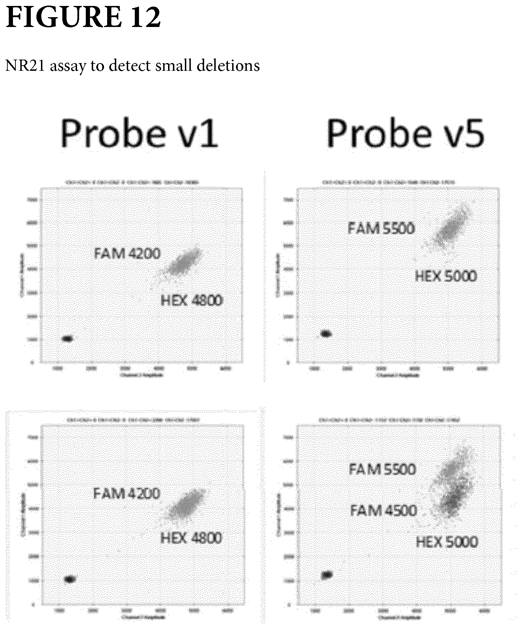

Figure 12 shows that the NR-21 assay designed to detect small differences in deletion length is effective in distinguishing a 2 nucleotide difference in the MSI marker between BT-549 (top two panels) and Daudi (bottom two panels) cell lines.

### DETAILED DESCRIPTION OF THE INVENTION

### I. Introduction

The present disclosure provides compositions and methods for detecting MSI with reduced risk of contamination, lower cost, and streamlined process compared to current methods. The presently disclosed methods use drop-off probes designed to bind to a normal genomic microsatellite repeat sequence that will be hydrolyzed (cleaved, fragmented) upon amplification of the microsatellite sequence if the drop-off probe is bound. The cleavage separates the label and quencher attached to the drop-off probe so that signal is generated. In the event of MSI, the drop-off probe will not bind as effectively to the microsatellite sequence, will not be cleaved to the same extent, and will not generate as much signal.

### II. Definitions

The term "biomarker" can refer to any detectable marker used to differentiate individual samples, *e.g.,* cancer versus non-cancer samples. Biomarkers include modifications (*e.g.*, methylation of DNA, phosphorylation of protein), differential expression, and mutations or variants (*e.g.*, single nucleotide variations, insertions, deletions, splice variants, and fusion variants). A biomarker can be detected in a DNA, RNA, and/or protein sample.

The term "multiplex" refers to an assay in which more than one target is detected, *e.g.,* in the same tube, well, droplet, or microchamber.

The terms "receptacle," "vessel," "tube," "well," "chamber," "microchamber," etc. refer to a containing space that can hold reagents or an assay. If the receptacle is in a kit and holds reagents, or is being used for an amplification reaction, it can be closed or sealed to avoid contamination or evaporation. If the receptacle is being used for an assay, it can be open or accessible, at least during set up of the assay.

The terms "individually detected" or "individual detection," referring to a marker gene or marker gene product, indicates that each marker in a multiplex reaction is detected. That is, each marker is associated with a different label (detected by a differently labeled probe).

The terms "nucleic acid," "polynucleotide," and "oligonucleotide" refer to polymers of nucleotides (*e.g.*, ribonucleotides or deoxyribo-nucleotides) and includes naturally-occurring (*e.g.*, adenosine, guanidine, cytosine, uracil and thymidine), and non-naturally occurring (human-modified) nucleic acids. The term is not limited by length (*e.g.*, number of monomers) of the polymer. A nucleic acid may be single-stranded or double-stranded and will generally contain 5'-3' phosphodiester bonds, although in some cases, nucleotide analogs may have other linkages. Monomers are typically referred to as nucleotides. The term "non-natural nucleotide" or "modified nucleotide" refers to a nucleotide that contains a modified nitrogenous base, sugar or phosphate group, or that incorporates a non-natural moiety in its structure. Examples of non-natural nucleotides include LNA, dideoxynucleotides, biotinylated, aminated, deaminated, alkylated, benzylated and fluorophor-labeled nucleotides.

The terms "cell-free nucleic acids," "cell-free DNA," "cell-free RNA," and like terms in the context of the present disclosure refers to a non-tissue sample (*e.g*., liquid biopsy) from an individual that has been processed to largely remove cells. Examples of non-tissue samples include blood and blood components, urine, saliva, tears, mucus, etc.

"LNA" refers to Locked Nucleic Acid. LNA is a modified RNA nucleotide in which the ribose moiety is modified with an extra bridge connecting the 2' oxygen and 4' carbon. LNA nucleotides can be mixed with DNA or RNA residues in any position in an oligonucleotide and hybridize with DNA or RNA according to Watson-Crick base-pairing rules. The locked ribose conformation enhances hybridization properties (*e.g.*, increases melting temperatures).

The term "primer" refers to a short nucleic acid (an oligonucleotide) that acts as a point of initiation of polynucleotide strand synthesis by a nucleic acid polymerase under suitable conditions. Polynucleotide synthesis and amplification reactions typically include an appropriate buffer, dNTPs and/or rNTPs, and one or more optional cofactors, and are carried out at a suitable temperature. A primer typically includes at least one target-hybridized region that is at least substantially complementary to the target sequence (*e.g.*, having 0, 1, or 2 mismatches). For the purposes of the present disclosure, this region of is typically about 4 to about 10 nucleotides in length, *e.g.,* 5-8 nucleotides. A "primer pair" refers to a forward and reverse primer that are oriented in opposite directions relative to the target sequence, and that produce an amplification product in amplification conditions. The terms "forward" and "reverse" are assigned arbitrarily. One of ordinary skill in the art will understand that forward and reverse primers (primer pair) define the borders of an amplification product. In some embodiments, multiple primer pairs rely on a single common forward or reverse primer. For example, multiple allele-specific forward primers can be considered part of a primer pair with the same, common reverse primer, *e.g.,* if the multiple alleles are in close proximity to each other. A "set of primers" or "primer set" can refer to a primer pair, or more than one primer pair designed to work together in a single multiplex reaction.

As used herein, "probe" means any molecule that is capable of selectively binding to a specifically intended target biomolecule, for example, a nucleic acid sequence of interest that hybridizes to the probes. The probe is detectably labeled with at least one non-nucleotide moiety. In some embodiments, the probe is labeled with a fluorophore and quencher.

The words "complementary" or "complementarity" refer to the ability of a nucleic acid in a polynucleotide to form a base pair with another nucleic acid in a second polynucleotide. For example, the sequence A-G-T (A-G-U for RNA) is complementary to the sequence T-C-A (U-C-A for RNA). Complementarity may be partial, in which only some of the nucleic acids match according to base pairing, or complete, where all the nucleic acids match according to base pairing. A probe or primer is considered "specific for" a target sequence if it is at least partially complementary to the target sequence. Depending on the conditions, the degree of complementarity to the target sequence is typically higher for a shorter nucleic acid such as a primer (e.g., greater than 80%, 90%, 95%, or 98%) than for a longer sequence. In some embodiments, primers and/or probes are 100% complementary to the targeted sequence.

The term "specifically amplifies" indicates that a primer set amplifies a target sequence more than non-target sequence at a statistically significant level. The term "specifically detects" indicates that a probe will detect a target sequence more than non-target sequence at a statistically significant level. As will be understood in the art, specific amplification and detection can be determined using a negative control, *e.g.,* a sample that includes the same nucleic acids as the test sample, but not the target sequence or a sample lacking nucleic acids. For example, primers and probes that specifically amplify and detect a target sequence result in a Ct that is readily distinguishable from background (non-target sequence), *e.g.*, a Ct that is at least 2, 3, 4, 5, 5-10, 10-20, or 10-30 cycles less than background. The term "allele-specific" PCR refers to amplification of a target sequence using primers that specifically amplify a particular allelic variant of the target sequence. Typically, the forward or reverse primer includes the exact complement of the allelic variant at that position.

The terms "identical" or "percent identity," in the context of two or more nucleic acids, or two or more polypeptides, refer to two or more sequences or subsequences that are the same or have a specified percentage of nucleotides, or amino acids, that are the same (*e.g.*, about 60% identity, e.g., at least any of 65%, 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters, or by manual alignment and visual inspection. See e.g., the NCBI web site at ncbi.nlm.nih.gov/BLAST. Such sequences are then said to be "substantially identical." Percent identity is typically determined over optimally aligned sequences, so that the definition applies to sequences that have deletions and/or additions, as well as those that have substitutions. The algorithms commonly used in the art account for gaps and the like. Typically, identity exists over a region comprising a sequence that is at least about 8-25 amino acids or nucleotides in length, or over a region that is 50-100 amino acids or nucleotides in length, or over the entire length of the reference sequence.

The terms "isolate," "separate," "purify," and like terms are not intended to be absolute. For example, isolation of DNA or genomic DNA does not require 100% of non-DNA molecules to be removed. One of skill in the art will recognize an acceptable level of purity for a given situation.

The term "kit" refers to any manufacture (*e.g.*, a package or a container) including at least one reagent, such as a nucleic acid probe or probe pool or the like, for specifically amplifying, capturing, tagging/converting or detecting RNA or DNA as described herein.

The term "amplification conditions" refers to conditions in a nucleic acid amplification reaction (*e.g.,* PCR amplification) that allow for hybridization and template-dependent extension of the primers. The term "amplicon" or "amplification product" refers to a nucleic acid molecule that contains all or a fragment of the target nucleic acid sequence and that is formed as the product of in vitro amplification by any suitable amplification method. The term "generate an amplification product" when applied to primers, indicates that the primers, under appropriate conditions (*e.g.*, in the presence of a nucleotide polymerase and NTPs), will produce the defined amplification product. Various PCR conditions are described in PCR Strategies (Innis et al., 1995, Academic Press, San Diego, CA) at Chapter 14; PCR Protocols: A Guide to Methods and Applications (Innis et al., Academic Press, NY, 1990)

The term "amplification product" refers to the product of an amplification reaction. The amplification product includes the primers used to initiate each round of polynucleotide synthesis. An "amplicon" is the sequence targeted for amplification, and the term can also be used to refer to amplification product. The 5' and 3' borders of the amplicon are defined by the forward and reverse primers. A "reverse transcription product," "RT product," and like terms refers to a cDNA molecule produced by elongation of an RT primer on an RNA template by a polymerase with reverse transcriptase activity.

The terms "individual", "subject", and "patient" are used interchangeably herein. The individual can be pre-diagnosis, post-diagnosis but pre-therapy, undergoing therapy, or post-therapy. In the context of the present disclosure, the individual is typically seeking medical care.

The term "sample" or "biological sample" refers to any composition containing or presumed to contain nucleic acid. The term includes purified or separated components of cells, tissues, or blood, *e.g.,* DNA, RNA, proteins, cell-free portions, or cell lysates. The sample can be FFPET, *e.g.,* from a tumor or metastatic lesion. The sample can also be from frozen or fresh tissue, or from a liquid sample, *e.g.*, blood or a blood component (plasma or serum), urine, semen, saliva, sputum, mucus, semen, tear, lymph, cerebral spinal fluid, mouth/throat rinse, bronchial alveolar lavage, material washed from a swab, etc. Samples also may include constituents and components of *in vitro* cultures of cells obtained from an individual, including cell lines. The sample can also be partially processed from a sample directly obtained from an individual, *e.g.*, cell lysate or blood depleted of red blood cells. A tumor sample can include tissue from a tumor, or a sample that includes DNA from a tumor, *e.g.*, ctDNA in the blood of a cancer patient.

The term "obtaining a sample from an individual" means that a biological sample from the individual is provided for testing. The obtaining can be directly from the individual, or from a third party that directly obtained the sample from the individual.

The term "providing therapy for an individual" means that the therapy is prescribed, recommended, or made available to the individual. The therapy may be actually administered to the individual by a third party *(e.g.,* an in-patient injection), or by the individual herself.

A "control" sample or value refers to a value that serves as a reference, usually a known reference, for comparison to a test sample or test conditions. For example, a test sample can be taken from a test condition, *e.g.*, from an individual suspected of having cancer, and compared to samples from known conditions, *e.g.*, from a cancer-free individual (negative control), or from an individual known to have cancer or a target sequence of interest (positive control). In the context of the present disclosure, the test sample is typically from a cancer patient, *e.g.,* a tumor sample. A control can also represent an average value or a range gathered from a number of tests or results (*e.g.*, from a number of known MSS or known MSI individuals). A control can also be prepared for reaction conditions. For example, a control for the presence, quality, and/ or quantity of nucleic acid (*e.g.*, internal control) can include primers or probes that will detect a sequence known to be present in the sample *(e.g.,* a housekeeping gene such as beta actin, beta globin, glyceraldehyde 3-phosphate dehydrogenase (GAPDH), ribosomal protein L37 and L38, PPIase, EIF3, eukaryotic translation elongation factor 2 (eEF2), DHFR, or succinate dehydrogenase). In some embodiments, the internal control can be a sequence from a region of the same gene that is not commonly variant (*e.g.*, in a different exon). A known added polynucleotide, *e.g.*, having a designated length, can also be added. An example of a negative control is one free of nucleic acids, or one including primers or probes specific for a sequence that would not be present in the sample, *e.g.*, from a different species. One of skill will understand that the selection of controls will depend on the particular assay, *e.g.,* so that the control is cell type and organism-appropriate. One of skill in the art will recognize that controls can be designed for assessment of any number of parameters. For example, a control can be devised to compare therapeutic benefit based on pharmacological data *(e.g.,* half-life) or therapeutic measures (*e.g.*, comparison of benefit and/or side effects). Controls can be designed for in vitro applications. One of skill in the art will understand which controls are valuable in a given situation and be able to analyze data based on comparisons to control values. Controls are also valuable for determining the significance of data. For example, if values for a given parameter are widely variant in controls, variation in test samples will not be considered as significant.

The terms "label," "tag," "detectable moiety," and like terms refer to a composition detectable by spectroscopic, photochemical, biochemical, immunochemical, chemical, or other physical means. For example, useful labels include fluorescent dyes (fluorophores), luminescent agents, radioisotopes (e.g., ³²P, ³H), electron-dense reagents, or an affinity-based moiety, *e.g.,* a poly-A (interacts with poly-T) or poly-T tag (interacts with poly-A), a His tag (interacts with Ni), or a strepavidin tag (separable with biotin). One of skill will understand that a detectable label conjugated to a nucleic acid is not naturally occurring.

Unless defined otherwise, technical and scientific terms used herein have the same meaning as commonly understood by a person of ordinary skill in the art. See, *e.g.,* Lackie, DICTIONARY OF CELL AND MOLECULAR BIOLOGY, Elsevier (4th ed. 2007); Sambrook et al., MOLECULAR CLONING, A LABORATORY MANUAL, Cold Springs Harbor Press (Cold Springs Harbor, N.Y. 1989). The term "a" or "an" is intended to mean "one or more." The terms "comprise," "comprises," and "comprising," when preceding the recitation of a step or an element, are intended to mean that the addition of further steps or elements is optional and not excluded.

### III. Microsatellite Instability (MSI) and Mismatch Repair (MMR)

Microsatellites are short repeats of DNA (1-6 base pairs) dispersed throughout the genome. There is some normal variation in the length of microsatellites between individuals. Microsatellite instability (MSI), however, results in differences in the repeat length in one or both alleles in an individual. This is brought about by defects in the mismatch repair (MMR) system.

Detection of MSI has both predictive and prognostic value. MSI-High (MSI-H) tumors are more likely to respond to immune checkpoint therapy *(e.g.,* PD-1 or PD-L1 targeting drugs such as Keytruda). In addition, patients with MSI-H CRC have better survival rates.

A panel of five mononucleotide repeat markers is recommended for testing by the National Cancer Institute (Suraweera et al. (2002) Gastroenterology 123:1804; Umar et al. (2004) J. Natl. Cancer Inst. 96:261). A sample is called MSI-High (MSI-H), if two or more markers show a difference in the repeat length between the normal control sample and the tumor samples. Common MSI markers are shown in Figure 1 (Zhang et al. (2008) J. Mol. Diagnostics 10:301). Current methods for MSI detection use fluorescent primers amplifying the relevant region, followed by capillary electrophoresis (CE) to identify size difference between the normal and tumor samples. A kit for one-well, multiplex detection of five mononucleotide repeat markers - BAT-25, BAT-26, NR-21, NR-24 and MONO-27 (and two pentanucleotide markers to detect potential sample mixups or contamination) - is commercially available (Bacher et al. (2004) Disease Markers 20:237). Typical readout is shown in Figure 2. This method suffers from high cost, laborious protocol, contamination risk (due to open tube design and post PCR handling), and limited analytical sensitivity (10% of tumor cell content required). Moreover, results must be reviewed to eliminate artifact peaks caused by bleed-through, stutter, or CE spikes.

### IV. Nucleic acid samples

Samples for biomarker detection can be obtained from any source suspected of containing nucleic acid, *e.g.,* tissue (including tumor tissue or FFPET tissue), blood, skin, swab (*e.g.*, buccal, vaginal), urine, saliva, etc.

Methods for isolating nucleic acids from biological samples are known, *e.g.*, as described in Sambrook, and several kits are commercially available (*e.g.*, High Pure RNA Isolation Kit, High Pure Viral Nucleic Acid Kit, and MagNA Pure LC Total Nucleic Acid Isolation Kit, DNA Isolation Kit for Cells and Tissues, DNA Isolation Kit for Mammalian Blood, High Pure FFPET DNA Isolation Kit, available from Roche). In the context of the presently disclosed methods, genomic DNA can be collected and isolated.

### V. Amplification and detection

A nucleic acid sample, *e.g.*, comprising genomic DNA< can be used for detection and quantification, *e.g.*, using nucleic acid amplification,

In some embodiments, the target nucleic acid is amplified with a thermostable polymerase with both reverse transcriptase activity and DNA template-dependent activity. Exemplary enzymes include Tth DNA polymerase, the C. therm Polymerase system, and those disclosed in US20140170730 and US20140051126.

Probes for use as described herein can be labeled with a fluorophore and optionally a quencher (*e.g.*, TaqMan, LightCycler, Molecular Beacon, Scorpion, or Dual Labeled probes). Appropriate fluorophores include but are not limited to FAM, JOE, TET, Cal Fluor Gold 540, HEX, VIC, Cal Fluor Orang 560, TAMRA, Cyanine 3, Quasar 570, Cal Fluor Red 590, Rox, Texas Red, Cyanine 5, Quasar 670, and Cyanine 5.5. Appropriate quenchers include but are not limited to TAMRA (for FAM, JOE, and TET), DABCYL, and BHQ1-3.

In some embodiments, digital PCR (dPCR) can be used to detect microsatellite instability. For example, digital droplet PCR (ddPCR) can be used to determine absolute measurement of a target nucleic acid in a sample, even at very low concentrations. The dPCR method comprises the steps of digital dilution or droplet generation, PCR amplification, detection and (optionally) analysis. The partitioning step comprises generation of a plurality of individual reaction volumes (*e.g.*, droplets or partitions) each containing reagents necessary to perform nucleic acid amplification. The PCR amplification step comprises subjecting the partitioned volumes to thermocycling conditions suitable for amplification of the nucleic acid targets to generate amplicons. Detection comprises identification of those partitioned volumes that contain and do not contain amplicons or fluorescent signal. The analysis step comprises a quantitation that yields, *e.g.*, concentration, absolute amount or relative amount (as compared to another target) of the target nucleic acid in the sample. Commercially available dPCR systems are available, *e.g.,* from Bio-Rad and ThermoFisher. Descriptions of dPCR can be found, *e.g.,* in US20140242582; Kuypers et al. (2017) J Clin Microbiol 55:1621; and Whale et al. (2016) Biomol Detect Quantif 10:15.

PCR detection devices are known in the art and can be selected as appropriate for the selected labels. Detection devices appropriate for quantitative PCR include the **cobas^{®}** and Light Cycler^{®} systems (Roche), PRISM 7000 and 7300 real-time PCR systems (Applied Biosystems), etc. Six-channel detection is available on the CFX96 Real Time PCR Detection System (Bio-Rad) and Rotorgene Q (Qiagen), allowing for a higher degree of multiplexing.

### VI. MSI and treatment

The presently disclosed methods are useful for detecting MSI, and directing therapy for MSI-H tumors and Lynch Syndrome (genetically inherited mutations in mismatch repair genes). In some embodiments, targeted therapy is prescribed, provided, or administered to a patient based on the presence or absence of a disease associated biomarker such as at least one MSI. In particular, patients with MSI-H tumors (MSI positive at 2 or more microsatellites), can be prescribed, provided, or administered an immune checkpoint therapy, e.g., therapies that target PD-1 or PD-L1. Examples include pembrolizumab (Keytruda), nivolumab (Opdivo), atezolizumab (Tecentriq), avelumab (Bavencio), and druvalumab (Imfinzi).

Several drugs are designed specifically for patients with certain biomarker profiles that may be tested in combination with MSI (e.g., Tarceva and Tagrisso for certain EGFR mutations). New targeted therapies are being developed to address a number of specific mutations, thus one of skill in the art will be in the best position to select a targeted therapy for an individual at the time.

In addition, cancer patients can benefit from standard chemotherapy. Thus, in some embodiments, chemotherapy is prescribed, provided, or administered to the patient based on the presence or absence of a cancer-associated biomarker. This can include CHOP (cyclophosphamide; doxorubicin; vincristine; and prednisolone) or R-CHOP, which further includes rituximab and/or etoposide. The cocktail can be administered periodically for a set period of time, or until reduction in tumor size and/or symptoms are detected. For example, the CHOP or R-CHOP can be administered every 2 or 3 weeks.

In the case of MSI-H patients, survival rates have been found to be higher than those for MSS patients. In some embodiments, treatment is prescribed, provided, or administered to adjust for longer term survival, *e.g.*, to reduce side effects.

Regardless of which treatment is selected, it typically begins with a low dose so that side effects can be determined, and the dose increased, *e.g.*, until side effects appear or within the patient's tolerance, or until clinical benefit is observed.

### VII. Kits

Provided herein are kits for carrying out microsatellite instability (MSI).

In some embodiments, the kit comprises a sample collection vessel (*e.g.*, tube, vial, multi-well plate or multi-vessel cartridge).

In some embodiments, the kit includes reagents and/or components for nucleic acid purification. For example, the kit can include a lysis buffer (*e.g.*, comprising detergent, chaotropic agents, buffering agents, etc.), enzymes or reagents for denaturing proteins or other undesired materials in the sample *(e.g.,* proteinase K), enzymes to preserve nucleic acids *(e.g.,* DNase and/or RNase inhibitors). In some embodiments, the kit includes components for nucleic acid separation, e.g., solid or semi-solid matrices such as chromatography matrix, magnetic beads, magnetic glass beads, glass fibers, silica filters, etc. In some embodiments, the kit includes wash and/or elution buffers for purification and release of nucleic acids from the solid or semi-solid matrix. For example, the kit can include components from MagNA Pure LC Total Nucleic Acid Isolation Kit, DNA Isolation Kit for Mammalian Blood, High Pure or MagNA Pure RNA Isolation Kits (Roche), DNeasy or RNeasy Kits (Qiagen), PureLink DNA or RNA Isolation Kits (Thermo Fisher), etc.

In some embodiments, the kit includes reagents for detection of particular target nucleic acids, *e.g.,* target nucleic acids associated with MSI, cancer, or other disease. For example, the kit can include oligonucleotides that specifically bind to microsatellite sequence, sequences adjacent to microsatellite sequence, cancer-associated biomarkers such as certain miRNAs, mutations, or sequences known to have copy number variations in cancer. In some embodiments, the detection reagents are for qPCR, dPCR, or sequencing (Sanger or NGS).

The kit can further include reagents for amplification, *e.g.*, reverse transcriptase, DNA polymerase, dNTPs, buffers, and/or other elements (*e.g.*, cofactors or aptamers) appropriate for reverse transcription and/or amplification. Typically, the reagent mixture(s) is concentrated, so that an aliquot is added to the final reaction volume, along with sample (*e.g.*, RNA or DNA), enzymes, and/ or water. In some embodiments, the kit further comprises reverse transcriptase (or an enzyme with reverse transcriptase activity), and/or DNA polymerase (*e.g.*, thermostable DNA polymerase such as Taq, ZOS, and derivatives thereof).

In some embodiments, the kit further includes at least one control sample, *e.g.*, nucleic acids from an MSS sample (or pooled samples), or from a sample known to carry a target sequence (or pooled samples). In some embodiments, the kit includes a negative control, *e.g.*, lacking nucleic acids, or lacking the targeted nucleic acid sequence. In some embodiments, the kit further includes consumables, *e.g.*, plates or tubes for nucleic acid preparation, tubes for sample collection, or plates, tubes, or microchips for PCR or qRT-PCR. In some embodiments, the kit further includes instructions for use, reference to a website, or software.

### VIII. Examples

### Example 1: Design strategy for digital PCR detection of MSI

We sought to detect deletion of nucleotides of a mononucleotide repeat. For this, we designed probes and primers to detect the BAT25 and BAT26 markers, which are 25 and 27 mononucleotide repeats, respectively. MSI at this marker has a wide range of deletion, from 1-13 nucleotides. We used a drop off dPCR assay, *i.e.*, with a "drop-off" probe that binds wild type sequence, but has reduced affinity for sequence with an insertion or deletion. The drop-off probe is used in combination with a reference probe, designed to hybridize to an invariant genomic sequence adjacent or near to the microsatellite sequence. A schematic is shown in Figure 3. The figure shows the position of the drop-off probe and reference (non-microsatellite) probe, as well as the position of the fluorophore and quencher on the probe. In principle, the quencher prevents fluorescence when the probe is intact, but if bound probe is hydrolyzed (displaced and cleaved) by polymerase during PCR, the fluorophore provides signal.

Challenges include detection of only a small deletion (*e.g.,* 1-2 nucleotides) while still detecting normal variations in allele length, and DNA from damaged DNA (*e.g.*, from an FFPET sample). Using probe design as shown in Figure 3 to detect differences in BAT25 (22 vs 25 polyT (SEQ ID NOS 23 and 24, respectively)) and BAT26 markers (18 vs 27 polyT (SEQ ID NOS 25 and 26, respectively)), we found inefficient quenching, and high baseline. This is in part due to the rigidity of a mononucleotide probe, and inefficient quenching with the quencher is positioned at the opposite end of the probe. Results are shown in Figure 4. The graphs show signal from the drop-off probe fluorophore on the vertical axis and signal from the reference probe fluorophore on the horizontal axis. Wild-type (non-MSI) signal appears in the upper right cluster, while MSI (Jurkat) signal is below, in the lower right cluster. The top graph is for BAT25 while the bottom graph is for BAT26 (27T (SEQ ID NO: 26)).

### Example 2: Use of quencher oligonucleotide with drop-off probe

The drop-off probes for these markers include a long stretch of polyT, which forms a rigid structure. The quencher therefore does not come close enough to the fluorophore to effectively quench. To improve quenching of unbound probe, we added a polyA quenching oligonucleotide. The drop-off probe and quencher oligonucleotide are shown as SEQ ID NOs:1 and 2, respectively, below.
Drop-off probe: 5'-FAM-TGATTTTTTTTTTTTTTTTTTTTTTTTTGAG-BHQ2-3'
Quenching oligo: 3'-BHQ2-AAAAAAAAAAAAAAAAA₁₆₋₂₀-5'

In principle, the quencher oligo binds to uncleaved probe below 50°C (exemplary PCR annealing temperature) and the hybridization holds the extra quencher closer to the fluorophore closer to improve quenching. The complex melts above 50°C and thus does not interfere with probe binding to template under dPCR conditions. When the drop-off probe gets cleaved by polymerase, the 5'-FAM fragment is short and does not bind back to the polyA quencher oligo at room (or detection) temperature, thus signal remains.

As shown in Figure 4, the quencher oligo worked well to reduce background with the BAT26 microsatellite, but not BAT25. Reduction in the length of the polyA quencher oligo to 16 nucleotides showed effective quenching and reduction of background for both microsatellites.

### Example 3: Alternative quenching improvements

We designed alternative approaches to improving quenching of the drop-off probe, as shown in Figure 5. In these approaches, the reference probe partially overlaps the drop-off probe so that, at lower temperature, the reference probe hybridizes to the drop-off probe and quenches the fluorophore. Figure 5 shows that these approaches, with either a 16 or 12 nucleotide overlap, reduce background similar to that of a separate 16-mer quencher probe.

### Example 4: Hairpin probes

We also sought to use a hairpin structured probe as the drop-off probe. In this case, the intact probe forms a hairpin structure through intra-molecular base pairing, thus bringing the quencher closer to the fluorophore (*see, e.g.*, Figure 6 and SEQ ID NO:3). The indicated shaded nucleotides in SEQ ID NO:3 form the hairpin, and hybridize to template. The hairpin melts above 50°C, so that probe binds target and is hydrolyzed by the polymerase.

### Example 5: Internal quencher

Another approach to reduce background/ improve quenching of the drop-off probe is by moving the quencher closer on the probe to the fluorophore. Figure 7 shows the drop-off probe for the BAT25 marker (SEQ ID NO:4) and the drop-off probe for the BAT26 marker (SEQ ID NO:5) with the quencher positioned at the 3' end or internally. The left plots show results of the MSI assays for BAT25 (top) and BAT26 (bottom) with the 3' quencher, and the right plots show results of the MSI assays for BAT25 (top) and BAT26 (bottom) with the internal quencher. In both assays, the internal quencher was more effective for reducing background fluorescence from unhydrolyzed drop-off probe.

### Example 6: Detection of small insertion/ deletions

While most tumors show a shift in microsatellite repeat sequence of several nucleotides, a significant percentage have shifts of only 1 or 2 nucleotides. For example, CRC tumors average a shift of 6 nucleotides, but 12% of tumors tested display only 1 nucleotide shift. In EMC, the average shift is 3 nucleotides, but 76% of tested samples show only a 1 nucleotide shift.

We sought to determine if the present methods could be used to detect small nucleotide shifts. Figure 8 shows that this method was effective for distinguishing the signal from several different MSI cell lines with deletions of various sizes.

### Example 7: Detection of MSI in FFPET samples

DNA from formalin-fixed paraffin embedded tissue (FFPET) samples is often damaged and fragmented. As tumor tissue is often preserved as FFPET, our methods would ideally work even on damaged DNA.

We tested BAT25 and BAT26 MSI assays on DNA from a wild type (MSS) FFPET sample and from MSI FFPET samples. The results show that the presently described MSI assays work even on FFPET DNA.

### Example 8: Multiplex MSI detection

To further streamline our assays compared to current capillary electrophoresis methods, we sought to determine if the presently described MSI assays can be performed in the same dPCR reaction. We combined the drop-off and reference probes and amplification primers for BAT25 and BAT26 microsatellites. The multiplex assays were carried out on cell lines with MSI at both sites, RAJI and Jurkat.

Figure 10 shows that the probes and primers did not interfere with each other, and that the results were clear. The left and middle panels show each of the single-plex reactions, which reveal the presence of two alleles in each cell line. The duplex results are shown on the right, and reveal the same results.

### Example 9: Detection of 5 MSI markers with internal quencher probes

We designed digital PCR assays for five commonly detected mononucleotide repeat markers: BAT-25, BAT-26, NR-21, NR-24 and Mono-27. All five markers showed expected phenotype on 2D scattered plot (Figure 11) when tested against genomic DNA from MSS cell-line (BT-549, upper panel, positive cluster showed high fluorescence in both drop-off probe FAM and reference probe HEX channels) and MSI-H cell-line (RKO cell-line, lower panel, positive cluster showed high fluorescence only in the reference probe HEX channel).

The sequences of the drop-off probes for BAT-25 and BAT-26 are the same internal quencher probes shown in Figure 7 (SEQ ID NOs:4 and 5, respectively). SEQ ID NO:6 represents the sequence of the internal quencher drop-off probe for NR-21 (<FAM_Thr>CCTTTTTTT<BHQ_2>TTTTTTTTTTTTTTTAGCAAC<Phos>). SEQ ID NO:7 represents the sequence of the internal quencher drop-off probe for NR-24 (<FAM_Thr>TCCTATTTTTT<BHQ_2>TTTTTTTTTTTTTTTTTTGTGAG<Phos>). SEQ ID NO:8 represents the sequence of the internal quencher drop-off probe for Mono-27 (<FAM_Thr>ACTCTTTTTT<BHQ_2>TTTTTTTTTTTTTTTTTTTTTGAG<Phos>.

### Example 10: NR-21 assay to detect small difference in nucleotide deletion

Daudi cells have a heterozygous or homozygous 2 nucleotide shortening of the NR-21 MSI marker compared to BT549 cells. Drop-off probes sometimes do not distinguish such small differences in repeat length. The first drop-off probe designed to detect NR-21 did not adequately distinguish between the MSI marker from Daudi and BT549 cells, leading us to design a drop-off probe sensitive enough to distinguish the 2 nucleotide difference in the NR-21 MSI marker.

Figure 12 shows the comparison of results using Probe 1 and Probe 5 on DNA from BT549 cells (top two panels) and Daudi cells (bottom two panels. Probe 1 represents the first drop-off probe with sequence
<FAM_Thr>TGGCCTTTTTTT<BHQ_2>TTTTTTTTTTTTTTTAGCA<Phos> (SEQ ID NO:9). Probe 5 is shown above as SEQ ID NO:6. As shown in Figure 12, Probe 5 distinguishes the higher FAM signal for the wild type NR-21 copy from the lower FAM signal from the NR-21 copy with the deletion.

## Claims

1. A kit comprising:
a) a drop-off probe having a sequence complementary to a wild type microsatellite repeat sequence in genomic DNA with at least one nucleotide on the 5' end and at least one nucleotide on the 3' end of the wild type microsatellite repeat sequence, wherein the drop-off probe is attached to a first label and a quencher and wherein the quencher of the drop-off probe is attached at a site within ten nucleotides of the first label; and
b) a reference probe having a sequence that is complementary to a reference sequence in genomic DNA that is not microsatellite repeat sequence, wherein the reference probe is attached to a second label and a quencher,
c) a set of amplification primers positioned 5' and 3' of the binding sites of the drop-off probe and reference probe, said set of amplification primers being designed to amplify both binding sites of the drop-off probe and the reference probe.

2. The kit of claim 1, wherein the drop-off probe has at least three nucleotides on the 5' end and at least three nucleotides on the 3' end of the wild type microsatellite repeat sequence.

3. The kit of any one of claims 1 and 2, wherein the quencher of the drop-off probe is attached at a site internal to the microsatellite repeat sequence.

4. The kit of any one of claims 1 to 3, wherein the drop-off probe has at least four nucleotides on the 5' end of the microsatellite repeat sequence.

5. The kit of any one of claims 1 to 4, further comprising:
c) a quencher oligonucleotide complementary to the drop-off probe, wherein the quencher probe comprises a quencher.

6. The kit of any one of claims 1 to 5, wherein the drop-off probe forms a hairpin structure below 50°C.

7. The kit of any one of claims 1 to 6, wherein the reference probe comprises sequence complementary to at least six nucleotides of the drop-off probe and quencher at the 3' end.

8. The kit of any one of claims 1 to 7, wherein the kit comprises at least one additional drop-off probe having a sequence complementary to a different wild type microsatellite repeat sequence in genomic DNA with at least one nucleotide on the 5' end and at least one nucleotide on the 3' end of the different wild type microsatellite repeat sequence, wherein the at least one additional drop-off probe is attached to a different label and a quencher.

9. The kit of claim 8, wherein the kit comprises at least one additional reference probe having a sequence that is complementary to a different reference sequence in genomic DNA that is not microsatellite repeat sequence, wherein the at least one additional reference probe is attached to a different label and a quencher.

10. The kit of any one of claims 1 to 9, further comprising a thermostable DNA polymerase.

11. A method for detecting microsatellite instability comprising
a) contacting genomic DNA with:
i) a drop-off probe having a sequence complementary to a wild type microsatellite repeat sequence in genomic DNA with at least one nucleotide on the 5' end and at least one nucleotide on the 3' end of the wild type microsatellite repeat sequence, wherein the drop-off probe is attached to a first label and a quencher and wherein the quencher of the drop-off probe is attached at a site within ten nucleotides of the first label;
ii) a reference probe having a sequence that is complementary to a reference sequence in genomic DNA that is not microsatellite repeat sequence, wherein the reference probe is attached to a second label and a quencher; and
iii) a set of amplification primers positioned 5' and 3' of the binding sites of the drop-off probe and reference probe, said set of amplification primers being designed to amplify both binding sites of the drop-off probe and the reference probe;
b) carrying out dPCR to amplify and detect the binding of drop-off probe and reference probe to genomic DNA, and
c) detecting microsatellite instability when binding of the drop-off probe to genomic DNA is reduced compared to a control or threshold value.

12. The method of claim 11, wherein the method is carried out in multiplex with two or more drop-off probes, reference probes, and sets of amplification primers positioned to detect instability at two or more microsatellite repeat sequences.

13. A reaction mixture comprising:
a) a drop-off probe having a sequence complementary to a wild type microsatellite repeat sequence in genomic DNA with at least one nucleotide on the 5' end and at least one nucleotide on the 3' end of the wild type microsatellite repeat sequence, wherein the drop-off probe is attached to a first label and a quencher and wherein the quencher of the drop-off probe is attached at a site within ten nucleotides of the first label;
b) a reference probe having a sequence that is complementary to a reference sequence in genomic DNA that is not microsatellite repeat sequence, wherein the reference probe is attached to a second label and a quencher;
c) a set of amplification primers positioned 5' and 3' of the binding sites of the drop-off probe and reference probe, said set of amplification primers being designed to amplify both binding sites of the drop-off probe and the reference probe;
d) a thermostable DNA polymerase; and
e) genomic DNA.

14. The reaction mixture of claim 13 further comprising:
at least one additional drop-off probe having a sequence complementary to a different wild type microsatellite repeat sequence in genomic DNA with at least one nucleotide on the 5' end and at least one nucleotide on the 3' end of the different wild type microsatellite repeat sequence, wherein the at least one additional drop-off probe is attached to a different label and a quencher; and
at least one additional reference probe having a sequence that is complementary to a different reference sequence in genomic DNA that is not microsatellite repeat sequence,
wherein the at least one additional reference probe is attached to a different label and a quencher.

## Patentansprüche

1. Kit, umfassend:
a) eine Drop-off-Sonde mit einer Sequenz, die komplementär ist zu einer Wildtyp-Mikrosatellitenwiederholungssequenz in genomischer DNA, mit mindestens einem Nukleotid am 5'-Ende und mindestens einem Nukleotid am 3'-Ende der Wildtyp-Mikrosatellitenwiederholungssequenz, wobei die Drop-off-Sonde an eine erste Markierung und einen Quencher gebunden ist und wobei der Quencher der Drop-off-Sonde an einer Stelle innerhalb von zehn Nukleotiden von der ersten Markierung angebracht ist; und
b) eine Referenzsonde mit einer Sequenz, die komplementär ist zu einer Referenzsequenz in genomischer DNA, die keine Mikrosatellitenwiederholungssequenz ist, wobei die Referenzsonde an eine zweite Markierung und einen Quencher gebunden ist,
c) einen Satz von Amplifikationsprimern, die 5' und 3' der Bindungsstellen der Drop-off-Sonde und Referenzsonde positioniert sind, wobei der Satz von Amplifikationsprimern dafür ausgebildet ist, beide Bindungsstellen der Drop-off-Sonde und der Referenzsonde zu amplifizieren.

2. Kit nach Anspruch 1, wobei die Drop-off-Sonde mindestens drei Nukleotide am 5'-Ende und mindestens drei Nukleotide am 3'-Ende der Wildtyp-Mikrosatellitenwiederholungssequenz aufweist.

3. Kit nach einem der Ansprüche 1 und 2, wobei der Quencher der Drop-off-Sonde an einer Stelle innerhalb der Mikrosatellitenwiederholungssequenz angebracht ist.

4. Kit nach einem der Ansprüche 1 bis 3, wobei die Drop-off-Sonde mindestens vier Nukleotide am 5'-Ende der Mikrosatellitenwiederholungssequenz aufweist.

5. Kit nach einem der Ansprüche 1 bis 4, ferner umfassend:
c) ein Quencher-Oligonukleotid, das komplementär ist zu der Drop-off-Sonde, wobei die Quenchersonde einen Quencher umfasst.

6. Kit nach einem der Ansprüche 1 bis 5, wobei die Drop-off-Sonde unter 50 °C eine Haarnadelstruktur bildet.

7. Kit nach einem der Ansprüche 1 bis 6, wobei die Referenzsonde eine Sequenz, die komplementär ist zu mindestens sechs Nukleotiden der Drop-off-Sonde, und einen Quencher am 3'-Ende umfasst.

8. Kit nach einem der Ansprüche 1 bis 7, wobei der Kit mindestens eine zusätzliche Drop-off-Sonde mit einer Sequenz umfasst, die komplementär ist zu einer anderen Wildtyp-Mikrosatellitenwiederholungssequenz in genomischer DNA, mit mindestens einem Nukleotid am 5'-Ende und mindestens einem Nukleotid am 3'-Ende der anderen Wildtyp-Mikrosatellitenwiederholungssequenz, wobei die mindestens eine zusätzliche Drop-off-Sonde an eine andere Markierung und einen Quencher gebunden ist.

9. Kit nach Anspruch 8, wobei der Kit mindestens eine zusätzliche Referenzsonde mit einer Sequenz umfasst, die komplementär ist zu einer anderen Referenzsequenz in genomischer DNA, die keine Mikrosatellitenwiederholungssequenz ist, wobei die mindestens eine zusätzliche Referenzsonde an eine andere Markierung und einen Quencher gebunden ist.

10. Kit nach einem der Ansprüche 1 bis 9, ferner umfassend eine thermostabile DNA-Polymerase.

11. Verfahren zum Nachweisen von Mikrosatelliteninstabilität, umfassend
a) das Inkontaktbringen genomischer DNA mit:
i) einer Drop-off-Sonde mit einer Sequenz, die komplementär ist zu einer Wildtyp-Mikrosatellitenwiederholungssequenz in genomischer DNA, mit mindestens einem Nukleotid am 5'-Ende und mindestens einem Nukleotid am 3'-Ende der Wildtyp-Mikrosatellitenwiederholungssequenz, wobei die Drop-off-Sonde an eine erste Markierung und einen Quencher gebunden ist und wobei der Quencher der Drop-off-Sonde an einer Stelle innerhalb von zehn Nukleotiden von der ersten Markierung angebracht ist;
ii) einer Referenzsonde mit einer Sequenz, die komplementär ist zu einer Referenzsequenz in genomischer DNA, die keine Mikrosatellitenwiederholungssequenz ist, wobei die Referenzsonde an eine zweite Markierung und einen Quencher gebunden ist; und
iii) einem Satz von Amplifikationsprimern, die 5' und 3' der Bindungsstellen der Drop-off-Sonde und Referenzsonde positioniert sind, wobei der Satz von Amplifikationsprimern dafür ausgebildet ist, beide Bindungsstellen der Drop-off-Sonde und der Referenzsonde zu amplifizieren;
b) das Durchführen von dPCR zum Amplifizieren und Nachweisen des Bindens der Drop-off-Sonde und der Referenzsonde an genomische DNA, und
c) das Nachweisen von Mikrosatelliteninstabilität, wenn das Binden der Drop-off-Sonde an genomische DNA im Vergleich zu einer Kontrolle oder einem Schwellenwert reduziert ist.

12. Verfahren nach Anspruch 11, wobei das Verfahren mehrfach mit zwei oder mehr Drop-off-Sonden, Referenzsonden und Sätzen von positionierten Amplifikationsprimern durchgeführt wird, um Instabilität an zwei oder mehr Mikrosatellitenwiederholungssequenzen nachzuweisen.

13. Reaktionsgemisch, umfassend:
a) eine Drop-off-Sonde mit einer Sequenz, die komplementär ist zu einer Wildtyp-Mikrosatellitenwiederholungssequenz in genomischer DNA, mit mindestens einem Nukleotid am 5'-Ende und mindestens einem Nukleotid am 3'-Ende der Wildtyp-Mikrosatellitenwiederholungssequenz, wobei die Drop-off-Sonde an eine erste Markierung und einen Quencher gebunden ist und wobei der Quencher der Drop-off-Sonde an einer Stelle innerhalb von zehn Nukleotiden von der ersten Markierung angebracht ist;
b) eine Referenzsonde mit einer Sequenz, die komplementär ist zu einer Referenzsequenz in genomischer DNA, die keine Mikrosatellitenwiederholungssequenz ist, wobei die Referenzsonde an eine zweite Markierung und einen Quencher gebunden ist;
c) einen Satz von Amplifikationsprimern, die 5' und 3' der Bindungsstellen der Drop-off-Sonde und der Referenzsonde positioniert sind, wobei der Satz von Amplifikationsprimern dafür ausgebildet ist, beide Bindungsstellen der Drop-off-Sonde und der Referenzsonde zu amplifizieren;
d) eine thermostabile DNA-Polymerase; und
e) genomische DNA.

14. Reaktionsgemisch nach Anspruch 13, ferner umfassend:
mindestens eine zusätzliche Drop-off-Sonde mit einer Sequenz, die komplementär ist zu einer anderen Wildtyp-Mikrosatellitenwiederholungssequenz in genomischer DNA, mit mindestens einem Nukleotid am 5'-Ende und mindestens einem Nukleotid am 3'-Ende der anderen Wildtyp-Mikrosatellitenwiederholungssequenz, wobei die mindestens eine zusätzliche Drop-off-Sonde an eine andere Markierung und einen Quencher gebunden ist; und
mindestens eine zusätzliche Referenzsonde mit einer Sequenz, die komplementär ist zu einer anderen Referenzsequenz in genomischer DNA, die keine Mikrosatellitenwiederholungssequenz ist, wobei die mindestens eine zusätzliche Referenzsonde an eine andere Markierung und einen Quencher gebunden ist.

## Revendications

1. Kit comprenant :
a) une sonde drop-off ayant une séquence complémentaire d'une séquence répétée microsatellitaire de type sauvage dans l'ADN génomique avec au moins un nucléotide sur l'extrémité 5' et au moins un nucléotide sur l'extrémité 3' de la séquence répétée microsatellitaire de type sauvage, dans lequel la sonde drop-off est fixée à un premier marqueur et à un extincteur et dans lequel l'extincteur de la sonde drop-off est fixé à un site dans la limite de dix nucléotides du premier marqueur ; et
b) une sonde de référence ayant une séquence qui est complémentaire d'une séquence de référence dans l'ADN génomique qui n'est pas une séquence répétée microsatellitaire, dans lequel la sonde de référence est fixée à un second marqueur et à un extincteur,
c) un ensemble d'amorces d'amplification positionnées en 5' et 3' des sites de liaison de la sonde drop-off et de la sonde de référence, ledit ensemble d'amorces d'amplification étant conçu pour amplifier les deux sites de liaison de la sonde drop-off et de la sonde de référence.

2. Kit selon la revendication 1, dans lequel la sonde drop-off a au moins trois nucléotides sur l'extrémité 5' et au moins trois nucléotides sur l'extrémité 3' de la séquence répétée microsatellitaire de type sauvage.

3. Kit selon l'une quelconque des revendications 1 et 2, dans lequel l'extincteur de la sonde drop-off est fixé au niveau d'un site interne à la séquence répétée microsatellitaire.

4. Kit selon l'une quelconque des revendications 1 à 3, dans lequel la sonde drop-off a au moins quatre nucléotides sur l'extrémité 5' de la séquence répétée microsatellitaire.

5. Kit selon l'une quelconque des revendications 1 à 4, comprenant en outre :
c) un oligonucléotide extincteur complémentaire de la sonde drop-off, dans lequel la sonde extinctrice comprend un extincteur.

6. Kit selon l'une quelconque des revendications 1 à 5, dans lequel la sonde drop-off forme une structure en épingle à cheveux au-dessous de 50 °C.

7. Kit selon l'une quelconque des revendications 1 à 6, dans lequel la sonde de référence comprend une séquence complémentaire d'au moins six nucléotides de la sonde drop-off et un extincteur à l'extrémité 3'.

8. Kit selon l'une quelconque des revendications 1 à 7, dans lequel le kit comprend au moins une sonde drop-off supplémentaire ayant une séquence complémentaire d'une séquence répétée microsatellitaire de type sauvage différente dans l'ADN génomique avec au moins un nucléotide sur l'extrémité 5' et au moins un nucléotide sur l'extrémité 3' de la séquence répétée microsatellitaire de type sauvage différente, dans lequel ladite sonde drop-off supplémentaire est fixée à un marqueur différent et à un extincteur.

9. Kit selon la revendication 8, dans lequel le kit comprend au moins une sonde de référence supplémentaire ayant une séquence qui est complémentaire d'une séquence de référence différente dans l'ADN génomique qui n'est pas une séquence répétée microsatellitaire, dans lequel l'au moins une sonde de référence supplémentaire est fixée à un marqueur différent et à un extincteur.

10. Kit selon l'une quelconque des revendications 1 à 9, comprenant en outre une ADN polymérase thermostable.

11. Procédé de détection d'une instabilité microsatellitaire comprenant
a) la mise en contact de l'ADN génomique avec :
i) une sonde drop-off ayant une séquence complémentaire d'une séquence répétée microsatellitaire de type sauvage dans l'ADN génomique avec au moins un nucléotide sur l'extrémité 5' et au moins un nucléotide sur l'extrémité 3' de la séquence répétée microsatellitaire de type sauvage, dans lequel la sonde drop-off est fixée à un premier marqueur et à un extincteur et dans lequel l'extincteur de la sonde drop-off est fixé à un site dans la limite de dix nucléotides du premier marqueur ;
ii) une sonde de référence ayant une séquence qui est complémentaire d'une séquence de référence dans l'ADN génomique qui n'est pas une séquence répétée microsatellitaire, dans lequel la sonde de référence est fixée à un second marqueur et à un extincteur ; et
iii) un ensemble d'amorces d'amplification positionnées en 5' et 3' des sites de liaison de la sonde drop-off et de la sonde de référence, ledit ensemble d'amorces d'amplification étant conçu pour amplifier les deux sites de liaison de la sonde drop-off et de la sonde de référence ;
b) la mise en oeuvre d'une dPCR pour amplifier et détecter la liaison de la sonde drop-off et de la sonde de référence à l'ADN génomique, et
c) la détection d'une instabilité microsatellitaire lorsque la liaison de la sonde drop-off à l'ADN génomique est réduite par comparaison à une valeur de contrôle ou seuil.

12. Procédé selon la revendication 11, dans lequel le procédé est mis en oeuvre en multiplex avec deux sondes drop-off ou plus, des sondes de référence et des ensembles d'amorces d'amplification positionnées pour détecter une instabilité au niveau de deux séquences répétées microsatellitaires ou plus.

13. Mélange réactionnel comprenant :
a) une sonde drop-off ayant une séquence complémentaire d'une séquence répétée microsatellitaire de type sauvage dans l'ADN génomique avec au moins un nucléotide sur l'extrémité 5' et au moins un nucléotide sur l'extrémité 3' de la séquence répétée microsatellitaire de type sauvage, dans lequel la sonde drop-off est fixée à un premier marqueur et à un extincteur et dans lequel l'extincteur de la sonde drop-off est fixé à un site à pas plus de dix nucléotides du premier marqueur ;
b) une sonde de référence ayant une séquence qui est complémentaire d'une séquence de référence dans l'ADN génomique qui n'est pas une séquence répétée microsatellitaire, dans lequel la sonde de référence est fixée à un second marqueur et à un extincteur ;
c) un ensemble d'amorces d'amplification positionnées en 5' et 3' des sites de liaison de la sonde drop-off et de la sonde de référence, ledit ensemble d'amorces d'amplification étant conçu pour amplifier les deux sites de liaison de la sonde drop-off et de la sonde de référence ;
d) une ADN polymérase thermostable ; et
e) un ADN génomique.

14. Mélange réactionnel selon la revendication 13 comprenant en outre :
au moins une sonde drop-off supplémentaire ayant une séquence complémentaire d'une séquence répétée microsatellitaire de type sauvage différente dans l'ADN génomique avec au moins un nucléotide sur l'extrémité 5' et au moins un nucléotide sur l'extrémité 3' de la séquence répétée microsatellitaire de type sauvage différente, dans lequel l'au moins une sonde drop-off supplémentaire est fixée à un marqueur différent et à un extincteur ; et
au moins une sonde de référence supplémentaire ayant une séquence qui est complémentaire d'une séquence de référence différente dans l'ADN génomique qui n'est pas une séquence répétée microsatellitaire, dans lequel l'au moins une sonde de référence supplémentaire est fixée à un marqueur différent et à un extincteur.
